# EUROPEAN PATENT APPLICATION

(11) **EP 3 919 825 A1**
(43) Date of publication of application: **08.12.2021**
(21) Application number: 19913624.3
(22) Date of filing: 29.05.2019
(51) Int. Cl.: F24F 3/16, F24F 13/28

(54) **AIR FILTRATION AND STERILIZATION DEVICE**

(30) Priority: 30.01.2019 CN 201910095886
(71) Applicant: Godox Photo Equipment Co., Ltd., Tangwei Community, Baoan District Shenzhen, Guangdong 518000 (CN)
(72) Inventor: ZENG, Weijun, Shenzhen, Guangdong 518000 (CN)
(74) Representative: Studio Torta S.p.A.
(86) International application number: PCT/CN2019/089111
(87) International publication number: WO 2020/155489

(57) **Abstract**

Disclosed is an air filtration and sterilization device, comprising a main housing, wherein an airflow channel for airflow circulation is provided inside the main housing, a filtration assembly is arranged at an air inlet end of the airflow channel, a fan (200) is connected to an air outlet end of the airflow channel, a gas receiving cavity (100) is connected between the filtration assembly and the fan (200), and a pulse flashing apparatus (300) is arranged in the gas receiving cavity (100). The pulse flashing apparatus (300) comprises a charging circuit (301) connected to an external power supply. The current output from the charging circuit (301) passes through a capacitor (302) and a discharging circuit (303) in sequence and then is input into an insert gas bulb (304). The sterilization device uses instantaneous high-strength pulsed light energy to kill various microorganisms with a short sterilization treatment time, and is particularly suitable for a working environment with a large gas flow.

## Description

### TECHNICAL FIELD

The present invention relates to furniture and home appliances, and more particularly to an air filtration and sterilization device.

### BACKGROUND

With the continuous improvement of people's living standards, people have put forward higher requirements for environmental health, and thus, air filters have also been widely adopted. The air filter is a device for purifying the air by trapping dust from the gas-solid two-phase flow through the function of porous filter materials, which is usually adopted in places such as biopharmaceuticals, hospitals, airport terminals, and human settlements. In order to better kill bacteria in the air, some air filters on the market today sterilize the air through chemicals or ultraviolet light.

### SUMMARY

### TECHNICAL PROBLEM

When the chemical medicine is used for sterilization, the chemical residue is likely to cause secondary pollution. When the ultraviolet light is used for killing bacteria passing through the air filter, the personnel need to be evacuated during work. Moreover, the efficiency of ultraviolet light sterilization is relatively low, and the air flow rate inside the air filter is relatively fast, which leads to a non-ideal sterilization effect.

### TECHNICAL SOLUTION

The purpose of the present invention is achieved through the following technical solutions.

The present invention provides an air filtration and sterilization device, comprising: a main housing; wherein an air flow path is provided in the main housing; a filter assembly, a fan, and a pulse flashing unit are provided on the air flow path; the pulse flashing unit comprises a charging circuit connected to an external power supply; a current output by the charging circuit passes through a capacitor and a discharging circuit in sequence, and then inputs to an inert gas lamp.

### BENEFICIAL EFFECTS

The air filtration and sterilization device provided in the present invention is equipped with a pulse flashing unit in the air flow path, such that bacteria in the air are killed through the strong light emitted by the pulse flashing unit. Therefore, the air filtration and sterilization device does not produce chemical residues, which also has a high sterilization efficiency and an ideal sterilization effect.

### BRIEF DESCRIPTION OF THE DRAWINGS

For ease of description, the present invention is described in detail with the following preferred embodiments and accompanying drawings.
FIG. 1 is an exploded structural diagram of an air filtration and sterilization device according to an embodiment of the present invention.
FIG. 2 is a cross-sectional structural diagram of the air filtration and sterilization device according to an embodiment of the present invention.
FIG. 3 is an overall structural diagram of the air filtration and sterilization device according to an embodiment of the present invention.
FIG. 4 is a schematic diagram of a circuit structure of a pulse flashing unit according to an embodiment of the present invention.
FIG. 5 is an exploded structural diagram of the air filtration and sterilization device according to another embodiment of the present invention.
FIG. 6 is a cross-sectional structural diagram of the air filtration and sterilization device according to another embodiment of the present invention.
FIG. 7 is an overall structural diagram of the air filtration and sterilization device according to another embodiment of the present invention.

### DETAILED DESCRIPTION

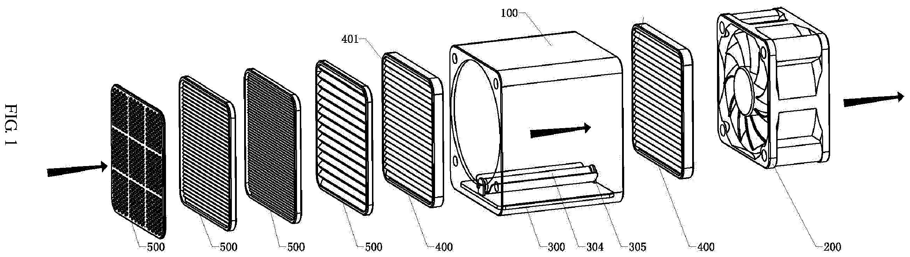

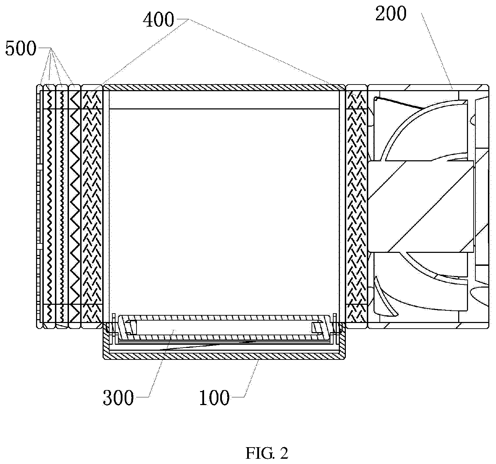

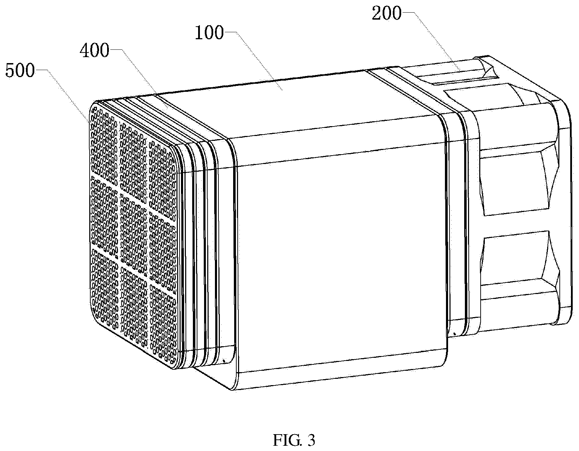

Referring to Figs. 1-4, the following embodiment specifically describes an air filtration and sterilization device, including: a main housing, and the main housing is provided with an air flow path for air to circulate. The air inlet end of the air flow path is provided with a filter assembly, and the air outlet end of the air flow path is connected to a fan 200. An air containing cavity 100 is provided between the filter assembly and the fan 200, and a pulse flashing unit 300 is provided in the air containing cavity 100. The pulse flashing unit 300 includes a charging circuit 301 connected to an external power supply, and the current output by the charging circuit 301 passes through a capacitor 302 and a discharging circuit 303 in sequence, and then inputs to the inert gas lamp 304. The external power supply is a mains supply or a battery. The working principle is specifically described as follows. When the power of the air filtration and sterilization device is turned on, the fan 103 runs, and the external air passes through the filter assembly and enters into the air containing cavity 100 under the action of the fan 103. The pulse flashing unit 300 in the air containing cavity 100 generates strong white light to irradiate the air in the air containing cavity 100 to kill bacteria therein, and finally the irradiated air is finally output by the fan 103. Therefore, the process of filtering and sterilizing the air is completed.

The working principle of the pulse flashing unit 300 is described as follows. The charging circuit 301 receives the current input from the external power supply and charges the capacitor 302. When the capacitor 302 is fully charged, the discharging circuit 303 drives the capacitor 302 to release a large current in an instant to drive the inert gas lamp 304 to emit a brief and intense white light.

In the embodiment, the pulsed strong light sterilization technology is adopted to effectively sterilize the air passing through the air containing cavity 100. The pulsed strong light sterilization technology is a world-leading light source technology, in which the pulse engineering technology for instantaneous discharging and a special inert gas lamp are adopted to stimulate strong white light in the form of pulses. The spectral distribution of the strong white light is similar to sunlight, and the light intensity thereof is equivalent to thousands or even tens of thousands of times the intensity of sunlight reaching the surface of the earth. In the pulsed strong light sterilization technology, instantaneous and high-intensity pulsed light energy is adopted to kill all kinds of microorganisms, thereby making up for the shortcomings of traditional heat sterilization and chemical sterilization. Moreover, due to the characteristics of short sterilization treatment time, the pulsed strong light sterilization technology is especially suitable for the working environment of air filtration sterilization equipment with high air fluidity.

Referring to Figs. 1-4, the following embodiment specifically describes an air filtration and sterilization device, including: a main housing, and the main housing is provided with an air flow path for air to circulate. The air inlet end of the air flow path is provided with a filter assembly, and the air outlet end of the air flow path is connected to a fan 200. An air containing cavity 100 is provided between the filter assembly and the fan 200, and a pulse flashing unit 300 is provided in the air containing cavity 100. The pulse flashing unit 300 includes a charging circuit 301 connected to an external power supply, and the current output by the charging circuit 301 passes through a capacitor 302 and a discharging circuit 303 in sequence, and then inputs to the inert gas lamp 304. The external power supply is a mains supply or a battery. The working principle is specifically described as follows. When the power of the air filtration and sterilization device is turned on, the fan 103 runs, and the external air passes through the filter assembly and enters into the air containing cavity 100 under the action of the fan 103. The pulse flashing unit 300 in the air containing cavity 100 generates strong white light to irradiate the air in the air containing cavity 100 to kill bacteria therein, and the irradiated air is finally output by the fan 103. Therefore, the process of filtering and sterilizing the air is completed.

The working principle of the pulse flashing unit 300 is described as follows. The charging circuit 301 receives the current input from the external power supply and charges the capacitor 302. When the capacitor 302 is fully charged, the discharging circuit 303 drives the capacitor 302 to release a large current in an instant to drive the inert gas lamp 304 to emit a brief and intense white light.

In the present invention, the pulsed strong light sterilization technology is adopted to effectively sterilize the air passing through the air containing cavity 100. The pulsed strong light sterilization technology is a world-leading light source technology, in which the pulse engineering technology for instantaneous discharging and a special inert gas lamp are adopted to stimulate strong white light in the form of pulses. The spectral distribution of the strong white light is similar to sunlight, and the light intensity thereof is equivalent to thousands or even tens of thousands of times the intensity of sunlight reaching the surface of the earth. In the pulsed strong light sterilization technology, instantaneous and high-intensity pulsed light energy is adopted to kill all kinds of microorganisms, thereby making up for the shortcomings of traditional heat sterilization and chemical sterilization. Moreover, due to the characteristics of short sterilization treatment time, the pulsed strong light sterilization technology is especially suitable for the working environment of air filtration sterilization equipment with high air fluidity.

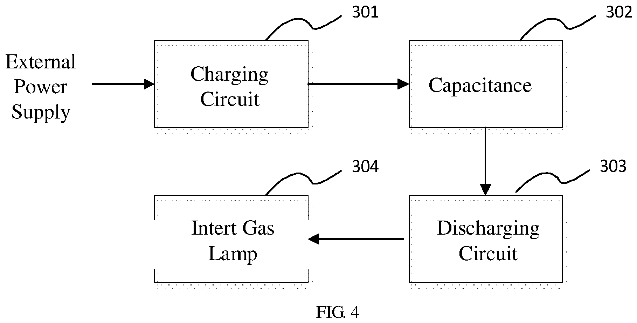

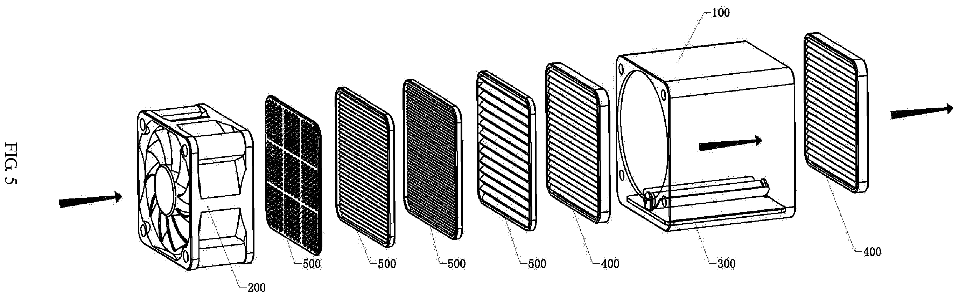

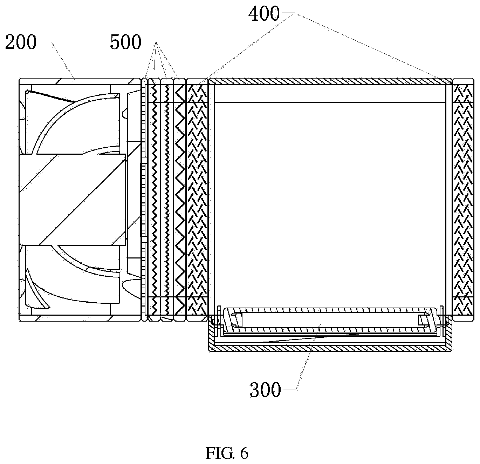

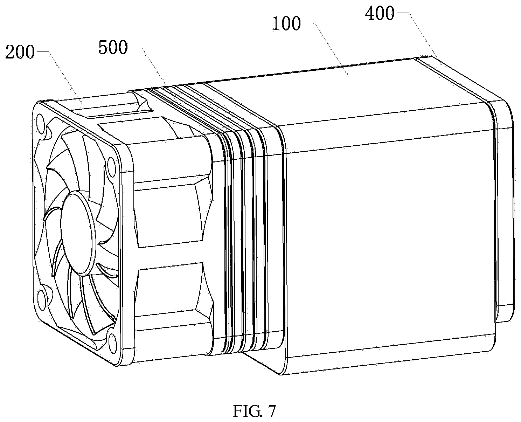

Referring to Figs. 4-7, the following embodiment specifically describes an air filtration and sterilization device, including: a main housing, and the main housing is provided with an air flow path for air to circulate. The air inlet end of the air containing cavity 100 is provided with a fan 200, and a rear end of the fan 200 is provided with a filter assembly. The air containing cavity 100 is provided at the air outlet end of the air flow path, and a pulse flashing unit 300 is provided in the air containing cavity 100. The pulse flashing unit 300 includes a charging circuit 301 connected to an external power supply, and the current output by the charging circuit 301 passes through a capacitor 302 and a discharging circuit 303 in sequence, and then inputs to the inert gas lamp 304. The external power supply is a mains supply or a battery. The working principle is specifically described as follows. When the power of the air filtration and sterilization device is turned on, the fan 103 runs, and the external air passes through the filter assembly and enters into the air containing cavity 100 under the action of the fan 103. The pulse flashing unit 300 in the air containing cavity 100 generates strong white light to irradiate the air in the air containing cavity 100 to kill bacteria therein, and the irradiated air is finally output by the air outlet end of the air containing cavity 100.

In the present invention, each of the air inlet end and the air outlet end of the air containing cavity 100 is covered with a light-blocking assembly 400, and the light-blocking assembly 400 is composed of more than two light barriers 401, and a space for air to pass is provided between two adjacent light barriers 401. The light barriers 401 are inclined to a light emitting surface of the inert gas lamp 304. Two light-blocking assemblies 400 are arranged between the filter assembly and the fan 103, to effectively prevent the strong light from leaking out of the main housing and causing damage to the user's eyes. A louvered structure is formed between two adjacent light barriers 401, which can effectively ensure that the air can smoothly pass through the gas containing cavity 100 while preventing the leakage of strong light.

In the present invention, the number of the pulse flashing unit 300 is more than one, and the pulse flashing unit 300 is fixedly arranged on the inner side wall of the air containing cavity 100. Specifically, the number of the pulse flashing units 300 can be increased or decreased according to actual conditions. When the air circulation speed is relatively fast, the number of the pulse flashing units 300 can be set to two, and each pulse flashing unit 300 flashes in sequence, thereby effectively shortening the flash cycle.

In the embodiment, a lamp shade 305 is provided on the outside of the inert gas lamp 304, and the lamp shade 305 is used to adjust the direction of light emission, thereby effectively improving the working efficiency of the product.

In the present invention, the surface of the light barrier 401 is coated with a light-absorbing material. Specifically, the light-absorbing material refers to a material that absorbs light and then diffusely reflects part of the light. When light shines on the light-absorbing material, there is no transmission outside of the illumination, nor does it produce mapping, large flares and reflections.

In the embodiment, the filter assembly is composed of more than two layers of filter screens 500. Through the cooperation of the multi-layer filter screen 500, the filter assembly can obtain a better filtering effect. Specifically, each filter screen 500 can have the same or different filtering function. The filter screen 500 can include: a pre-filter, an ozone-removing filter, an activated carbon filter, a first high efficiency particulate air (HEPA) filter, a formaldehyde removal filter, a sterilization filter, a second high efficiency particulate air (HEPA) filter and other air purification filters.

In the present invention, the inert gas lamp 304 is a xenon lamp. The xenon lamp refers to a bulb filled with an inert gas mixture including xenon.

In the description of this specification, the description with reference to the terms "one embodiment", "some embodiments", "exemplary embodiments", "examples", "specific examples" or "some examples" means that the specific feature, structure, material or characteristic described in combination with the embodiment or example is included in at least one embodiment or example of the present invention. Moreover, the described specific features, structures, materials or characteristics can be combined in an appropriate manner in any one or more embodiments or examples.

Described above are only the preferred embodiments of the present invention and are not intended to limit the present invention. Any modification, equivalent replacement and improvement made within the spirit and principle of the present invention shall be included in the protection scope of the present invention.

### Industrial applicability

The air filtration and sterilization device of the present invention can be applied to various densely populated scenes, which has extremely high industrial applicability.

## Claims

1. An air filtration and sterilization device, comprising:
a main housing;
wherein an air flow path is provided in the main housing; a filter assembly, a fan, and a pulse flashing unit are provided on the air flow path; the pulse flashing unit comprises a charging circuit connected to an external power supply; a current output by the charging circuit passes through a capacitor and a discharging circuit in sequence, and then inputs to an inert gas lamp.

2. The air filtration and sterilization device of claim 1, wherein the filter assembly is arranged on an air inlet end of the air flow path; the fan is arranged on an air outlet end of the air flow path; and the pulse flashing unit is arranged between the filter assembly and the fan.

3. The air filtration and sterilization device of claim 1, wherein the fan is arranged on an air inlet end of the air flow path; the filter assembly is arranged at a rear end of the fan; and the pulse flashing unit is arranged at a rear end of the filter assembly.

4. The air filtration and sterilization device of claim 2 or claim 3, wherein the pulse flashing unit is arranged in an air containing cavity; the number of the pulse flashing unit in the air containing cavity is at least one; and the pulse flashing unit is fixedly arranged on an inner side wall of the air containing cavity.

5. The air filtration and sterilization device of claim 4, wherein an outer side of the inert gas lamp is provided with a lamp shade.

6. The air filtration and sterilization device of claim 5, wherein each of the air inlet end and the air outlet end of the air containing cavity is covered with a light-blocking assembly; the light-blocking assembly is composed of more than two light barriers; a space for air to pass is provided between two adjacent light barriers; and the light barriers are inclined to a light emitting surface of the inert gas lamp.

7. The air filtration and sterilization device of claim 6, wherein a surface of each of the light barriers is coated with light-absorbing material.

8. The air filtration and sterilization device of claim 7, wherein the filter assembly is composed of more than two layers of filter screens.

9. The air filtration and sterilization device of claim 8, wherein the inert gas lamp is a xenon lamp.
